# EUROPEAN PATENT SPECIFICATION

(11) **EP 3 972 607 B1**
(45) Date of publication and mention of the grant of the patent: **25.02.2026**
(21) Application number: 20810176.6
(22) Date of filing: 15.04.2020
(51) Int. Cl.: A61K 31/522, A61K 39/395, A61P 35/00, C07D 473/08, A61K 45/06

(54) **PHARMACEUTICAL COMBINATION**
PHARMAZEUTISCHE KOMBINATION
COMBINAISON PHARMACEUTIQUE

(30) Priority: 22.05.2019 SG 10201904589X
(43) Date of publication of application: 30.03.2022
(73) Proprietor: Agency for Science, Technology and Research, Singapore 138632 (SG)
(72) Inventor: O'CONNELL, Damian, Singapore 138670 (SG); TENEGGI, Vincenzo, Singapore 138670 (SG); DIERMAYR, Veronica, Singapore 138670 (SG)
(74) Representative: AWA Sweden AB
(86) International application number: PCT/SG2020/050231
(87) International publication number: WO 2020/236078

(56) References cited:
- WO-A1-2014/189466
- WO-A1-2014/189466
- WO-A1-2018/117972
- WO-A1-2018/150312
- ANONYMOUS, 13 August 2015 (2015-08-13), pages 1 - 13, XP093057381, Retrieved from the Internet <URL:https://classic.clinicaltrials.gov/ct2/show/NCT02521844> [retrieved on 20230623]
- B MADAN ET AL: "Wnt addiction of genetically defined cancers reversed by PORCN inhibition", ONCOGENE, vol. 35, no. 17, 10 August 2015 (2015-08-10), London, pages 2197 - 2207, XP055496939, ISSN: 0950-9232, DOI: 10.1038/onc.2015.280
- ANONYMOUS: "A study to evaluate the safety and tolerability of ETC-1922159 in advanced solid tumours", CLINICAL TRIAL STUDY NCT02521844: STUDY DESCRIPTION PART B, CONDITIONS, ARMS AND INTERVENTIONS, 1 January 2019 (2019-01-01), pages 1 - 11, XP055762920, Retrieved from the Internet <URL:https://classic.clinicaltrials.gov/ct2/show/NCT02521844> [retrieved on 20210106]
- QIN A ET AL: "Clinical Predictors of Durable Clinical Benefit of Pembrolizumab Therapy in Veterans With Metastatic Non-Small Cell Lung Cancer", INTERNATIONAL JOURNAL OF RADIATION: ONCOLOGY BIOLOGY PHYSICS, vol. 98, no. 1, 2017, pages 228, XP029974716, ISSN: 0360-3016, DOI: 10.1016/J.IJROBP.2017.01.141
- DATABASE MEDLINE [online] US NATIONAL LIBRARY OF MEDICINE (NLM), BETHESDA, MD, US; April 2017 (2017-04-01), BILGIN BURAK ET AL: "Targeting the PD-1 pathway: a new hope for gastrointestinal cancers.", XP002809614, Database accession no. NLM28055269
- BILGIN BURAK ET AL: "Targeting the PD-1 pathway: a new hope for gastrointestinal cancers.", CURRENT MEDICAL RESEARCH AND OPINION 04 2017, vol. 33, no. 4, April 2017 (2017-04-01), pages 749 - 759, XP009544998, ISSN: 1473-4877, DOI: 10.1080/03007995.2017.1279132
- "A study to evaluate the safety and tolerability of ETC-1922159 in advanced solid tumours", CLINICAL TRIAL STUDY NCT02521844: STUDY DESCRIPTION PART B, CONDITIONS, ARMS AND INTERVENTIONS, 14 May 2019 (2019-05-14), pages 1 - 11, XP055762920, Retrieved from the Internet <URL:https://clinicaItrials.gov/ct2/history/NCT02521844?V_7=View#StudyPageTop> [retrieved on 20200930]
- BHAMRA, I. ET AL.: "Porcupine inhibitor RXC004 enhances immune response in pre-clinical models of cancer", ACCR, CANCER RESEARCH, vol. 78, no. 13, 1 July 2018 (2018-07-01), XP009516677, DOI: 10.1158/1538-7445. AM 2018-3764

## Description

### Field of Invention

The invention relates generally to the field of oncology. In particular, the invention relates to a pharmaceutical combination comprising a compound of formula (I) and an anti-PD-1 antibody for treating diseases and/or disorders related to the Wnt pathway.

### Background

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Cancer is typically treated with either chemotherapy and/or radiation therapy. Whilst often effective to destroy a significant amount of tumor cells, such therapies often leave behind a number of tumor cells that is resistant to the treatment. These resistant cells can proliferate to form new tumors that are then resistant to treatment. As a result, the constant use of known combinations of chemotherapeutic drugs has given rise to multidrug resistant ('MDR') tumor cells. WO2014189466 discloses compounds of formula (I) for use in modulation of the Wnt pathway to treat a disease or condition, which is a cancer.

The mode of proliferative diseases, such as tumors, is multi-factorial. For instance, research over the last forty years has led to the realization that cytotoxic agents (or anti-proliferative agents) includes anti-metabolic agents which interfere with microtubule formulation, alkylating agents which are able to cross-link DNA, platinum based agents which are able to interfere with DNA alkylation by blocking DNA replication, antitumor antibiotic agents, topoisomerase inhibitors, etc. In the treatment of such diseases drugs with different mechanisms may be combined (i.e., combination therapies) with beneficial effects including the effective treatment of MDR tumor cells and to minimize side effects such as undesirable cytotoxicity. The difficulty here is that not all known anti-proliferative agents provide useful or beneficial effects in combination and accordingly research in many laboratories is presently focused on developing new and useful anti-proliferative combination partners. Accordingly, it is generally desirable to produce a pharmaceutical combination which overcomes or ameliorate one or more of the above mentioned difficulties.

### Summary

The present invention provides a pharmaceutical combination, comprising:
a) a compound of Formula (I) having the following structure: 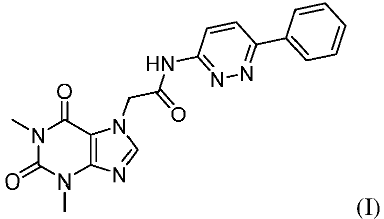 or a pharmaceutically acceptable salt or solvate thereof; and
b) and an anti-PD-1 antibody molecule or a pharmaceutically acceptable salt or solvate thereof.

In one embodiment, the anti-PD-1 antibody molecule is pembrolizumab.

Disclosed herein is a method of treating a proliferative disease or disorder associated with Wnt pathway in a subject, the method comprising administering a pharmaceutical combination as defined herein to the subject. The scope of the invention is defined by the claims. Any references in the description to methods of treatment refer to the compounds, pharmaceutical compositions and medicaments of the present invention for use in a method for treatment of the human or animal body by therapy.

In an embodiment, the proliferative disease or condition is colorectal cancer or endometrial cancer.

Disclosed herein is a pharmaceutical combination as defined herein for use in the treatment of a proliferative disease or disorder associated with Wnt pathway in a subject.

Disclosed herein is the use of a pharmaceutical combination as defined herein in the manufacture of a medicament for the treatment of a proliferative disease or disorder associated with Wnt pathway in a subject.

### Brief Description of Drawings

Embodiments of the present invention are hereafter described, with reference to the accompanying drawings in which:

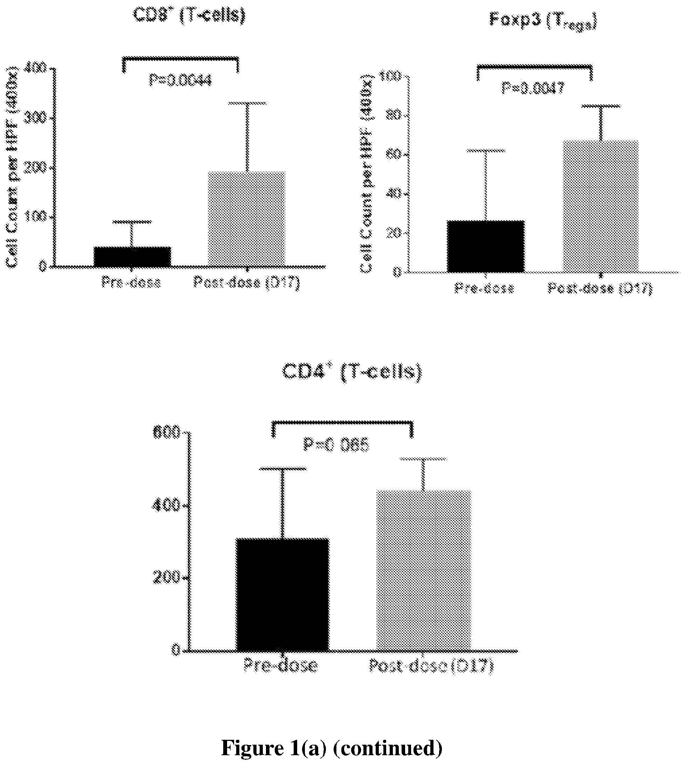

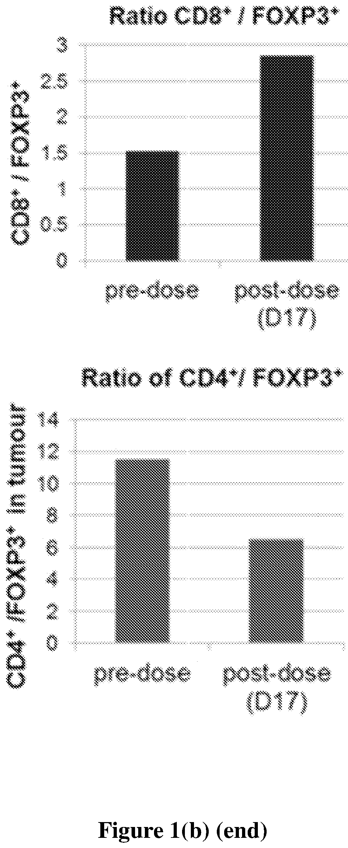

**Figure** 1: FFPE tumor tissue of patient 202-101 with unknown primary tumour (presumably cholangiocarcinoma) was prepared from paired biopsies, pre-dose and on C1D17 post-dose. Sections were assayed via IHC/OPAL and analysed on the Vectra3 (Perkin Elmer). The ratio of CD8⁺ to FOXP3⁺ and CD4⁺ to FOXP3⁺ cells was plotted with numbers determined from 3 high-powered fields measured on a single stained section.

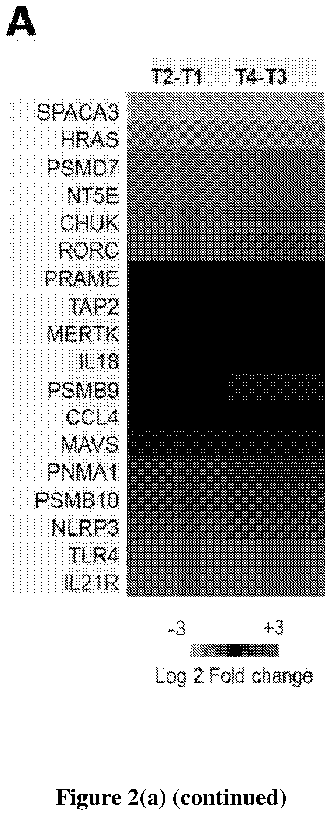

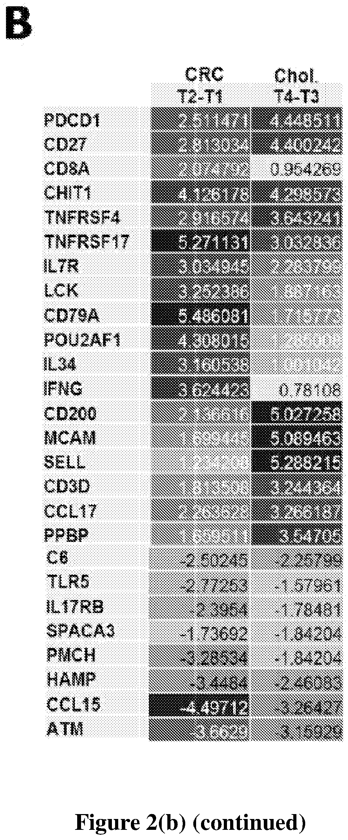

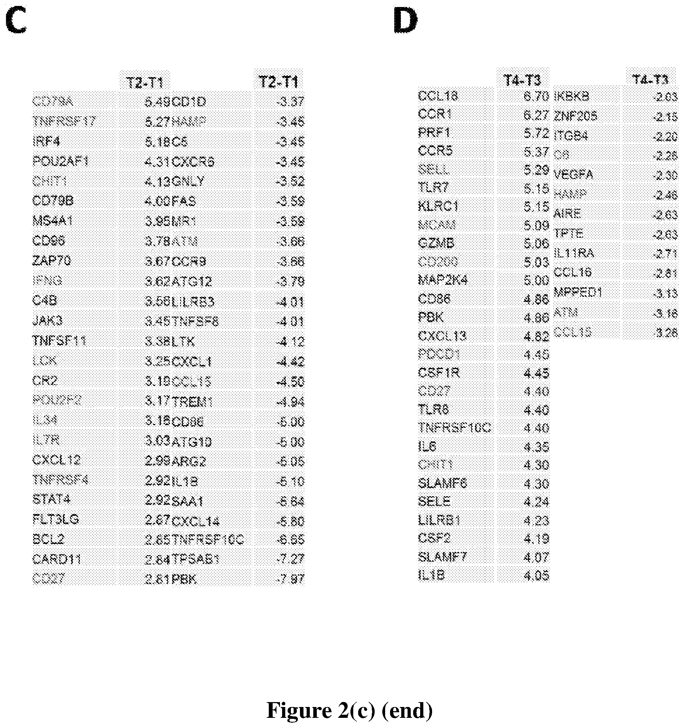

**Figure** 2 Nanostring analysis of FFPE and frozen tumor tissues from the patient with unknown primary (presumably cholangiocarcinoma) 202-101 (T4-T3) and the MSS CRC 102-102 (T2-T1) patient (bearing an RSPO gene fusion) using the Pan-Cancer immune panel. (A) Grouped analysis showed similar pre- and post-dose changes in both samples. (B) Most up- and downregulated genes common for both cancer types are shown. (C and D) Shows the most up and down-regulated genes in the CRC patient with RSPO fusion and the patient with unknown primary tumour (presumed to be cholangiocarcinoma), respectively. Red gene symbols indicate genes that are up- or down-regulated in both of the patients.

### Detailed Description

The present invention provides a pharmaceutical combination, comprising:
a) a compound of Formula (I) having the following structure: 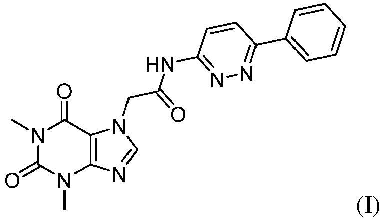 or a pharmaceutically acceptable salt or solvate thereof; and
b) and an anti-PD-1 antibody molecule or a pharmaceutically acceptable salt or solvate thereof.

Without being bound by the theory, the invention is predicated on the discovery that the compound of Formula (I) is compatible with an anti-PD-1 antibody molecule as a combination therapy for treating cancers (such as microsatellite stable (MSS) colorectal cancers or endometrial cancers). The inventors have found that the compound of Formula (I) (compound A) results in an increase in infiltrating lymphocytes in tumours and increased ratios of CD8⁺ to FOXP3⁺ T-cells. In addition, Nanostring analysis showed that genes such as PDCD1 and IFNG were highly upregulated while others such as checkpoint kinase ATM and chemokine CCL15 were highly downregulated. The increase in tumour-infiltrating lymphocytes (TILS) and the change in the tumour environment suggest that the tumour has changed from an immunological "cold" tumor to an immunologically "hot" tumor and can be further treated with an anti-PD-1 antibody molecule.

In the present disclosure the term "pharmaceutical combination" refers to a non-fixed combination. The term "non-fixed combination" means that the active ingredients, e.g. compound of formula (i), namely a compound of Formula (I) having the following structure: 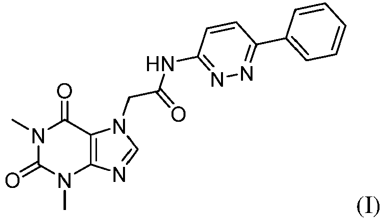 or a pharmaceutically acceptable salt or solvate thereof and an anti-PD-1 antibody molecule or a pharmaceutically acceptable salt or solvate thereof are both administered to a patient as separate entities either simultaneously or sequentially with no specific time limits, wherein such administration provides therapeutically effective levels of the two compounds in the body of the patient.

The terms "a combination" or "in combination with," it is not intended to imply that the therapy or the therapeutic agents must be administered at the same time and/or formulated for delivery together, although these methods of delivery are within the scope described herein. The therapeutic agents in the combination can be administered concurrently with, prior to, or subsequent to, one or more other additional therapies or therapeutic agents. The therapeutic agents or therapeutic protocol can be administered in any order. In general. each agent will be administered at a dose and/or on a time schedule determined for that agent. In will further be appreciated that the additional therapeutic agent utilized in this combination may be administered together or separately in different compositions. In general, it is expected that additional therapeutic agents utilized in combination be utilized at levels that do not exceed the levels at which they are utilized individually. In some embodiments, the levels utilized in combination will be lower than those utilized individually.

For example, the anti-PD-1 antibody molecule may be administered prior to administration of the compound of Formula (I). The anti-PD-1 antibody molecule may also be administered following administration of the compound of Formula (I). The compound of Formula (I) may also be administered concurrently with the anti-PD-1 antibody molecule. In one embodiment, the compound of Formula (I) is administered once every day (e.g. via oral administration) while the anti-PD-1 antibody molecule is administered once every 21 days (e.g. via intravenous infusion).

The combination partners. as described herein, can be synergistically active. while causing less side effects caused by the Wnt signaling pathway inhibition such as reduced bone density.

The term "pharmaceutically acceptable salts" can be formed, for example, as acid addition salts. preferably with organic or inorganic acids. Suitable inorganic acids are, for example. halogen acids, such as hydrochloric acid. Suitable organic acids are, e.g., carboxylic acids or sulfonic acids. such as fumaric acid or methanesulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations). and these are therefore preferred. Any reference to the free compound herein is to be understood as referring also to the corresponding salt, as appropriate and expedient. The salts of the inhibitors, as described herein, are preferably pharmaceutically acceptable salts; suitable counter-ions forming pharmaceutically acceptable salts are known in the field.

The term "pharmaceutically acceptable" refers to those compounds, materials, compositions. and/or dosage forms which are suitable for use in contact with the tissues of human beings and animals without excessive toxicity, irritation, allergic response, or other problem or complication, commensurate with a reasonable benefit/risk ratio.

The term "inhibition" or "inhibitor" includes a reduction in a certain parameter, e.g., an activity, of a given molecule, e.g., an immune checkpoint inhibitor, such as the anti-PD-1 antibody molecule. For example, inhibition of an activity, e.g., a PD-1 or PD-L1 activity, of at least 5%, 10%, 20%, 30%, 40% or more is included by this term. Thus, inhibition need not be 100%.

The term "antibody molecule" refers to a protein, e.g., an immunoglobulin chain or fragment thereof, comprising at least one immunoglobulin variable domain sequence. The term "antibody molecule" includes, for example, a monoclonal antibody (including a full length antibody which has an immunoglobulin Fc region). An antibody molecule comprises a full length antibody, or a full length immunoglobulin chain, or an antigen binding or functional fragment of a full length antibody, or a full length immunoglobulin chain. An antibody molecule can also be a multi-specific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope.

An antibody molecule can be derivatized or linked to another functional molecule (e.g., another peptide or protein). Methods of derivatization include but are not limited to the addition of a fluorescent moiety. a radionucleotide, a toxin. an enzyme or an affinity ligand such as biotin. Accordingly, the antibody molecules of the invention are intended to include derivatized and otherwise modified forms of the antibodies described herein, including immunoadhesion molecules. For example, an antibody molecule can be functionally linked (by chemical coupling, genetic fusion. noncovalent association or otherwise) to one or more other molecular entities. such as another antibody (e.g., a bispecific antibody or a diabody). a detectable agent, a cytotoxic agent, a pharmaceutical agent, and/or a protein or peptide that can mediate association of the antibody or antibody portion with another molecule (such as a streptavidin core region or a poly-histidine tag).

By "binding fragment" or "antigen-binding fragment" of an antibody, it is intended to indicate any peptide, polypeptide. or protein retaining the ability to bind to the target (also generally referred as antigen) of the antibody. In an embodiment, such "antigen binding fragments" are selected in the group consisting of Fv, scFv (sc for single chain), Fab, F(ab')₂, Fab', scFv-Fc fragments or diabodies, or any fragment of which the half- life time would have been increased by chemical modification, such as the addition of poly(alkylene) glycol such as poly(ethylene) glycol ("PEGylation") (pegylated fragments called Fv-PEG, scFv-PEG, Fab-PEG, F(ab')₂-PEG or Fab'-PEG) ("PEG" for Poly(Ethylene) Glycol), or by incorporation into a liposome. In one embodiment. the "antigen binding fragments" will be constituted or will comprise a partial sequence of the heavy or light variable chain of the antibody from which they are derived, said partial sequence being sufficient to retain the same specificity of binding as the antibody from which it is descended and a sufficient affinity, for example at least equal to 1/100, as a further example to at least equal to 1/10, of the affinity of the antibody from which it is desectided, with respect to the target.

The anti-PD-1 antibody molecule may be an anti-PD-1 antibody that is known in the art. In one embodiment, the anti-PD-1 antibody is MDX-1106 (also known as MDX-1106-04. ONO-4538. BMS-936558 or nivolumab). In one embodiment, the anti-PD-1 antibody is nivolumab (CAS Registry Number: 946414-94-4). Nivolumab (also referred to as BMS-936558 or MDX1106; Bristol-Myers Squibb) is a fully human IgG4 monoclonal antibody that specifically blocks PD-1. Nivolumab (clone 5C4) and other human monoclonal antibodies that specifically bind to PD-1 are disclosed in US 8.008.449 Patent and WO2006/121168. Ranburorizumabu (also called pembrolizumab or MK03475; Merck) is a humanized IgG4 monoclonal antibody that specifically binds to PD-1. Pembrolizumab is disclosed in US 8,354,509 Patent and WO2009/114335. In some embodiments, the anti-PD-1 antibody is pidilizumab. Pidilizumab (CT-011; Cure Tech) is a humanized IgGlk monoclonal antibody that binds to PD1. Pidilizumab and other humanized anti-PD-1 monoclonal antibodies are disclosed in WO2009/101611. In one embodiment, the anti-PD-1 antibody is PDR-001, which is a humanized monoclonal antibody described in WO2015/112900. Other anti- PD1 antibodies are disclosed in US 8,609,089, US 2010028330, and/or US 20120114649. Other anti-PD-1 antibodies include AMP 514 (Amplimmune).

The anti-PD-1 antibody molecule may be selected from nivolumab (Opdivo), pembrolizumab (Keytruda), pidilizumab or PDR-001. In one embodiment, the anti-PD-1 antibody molecule is pembrolizumab.

It is understood that the anti-PD-1 antibody molecule, or the anti-PD-1 antibody molecule, of the present disclosure may have additional conservative or non-essential amino acid substitutions, which do not have a substantial effect on their functions.

Disclosed herein is a method of treating a proliferative disease or disorder associated with Wnt pathway in a subject, the method comprising administering a pharmaceutical combination as defined herein to the subject. The pharmaceutical combination may be administered for a sufficient time and under conditions to treat the subject of the proliferative disease or disorder associated with the Wnt pathway.

The term "treatment" comprises, for example, the therapeutic administration of the combination of a Wnt inhibitor, or a pharmaceutically acceptable salt thereof, and an anti-PD-1 antibody molecule, or a pharmaceutically acceptable salt thereof, as described herein to a warm- blooded animal, in particular a human being, in need of such treatment with the aim to cure the disease or to have an effect on disease regression or on the delay of progression of a disease. The terms "treat", "treating" or "treatment" of any disease or disorder refers to ameliorating the disease or disorder [e.g. slowing or arresting or reducing the development of the disease or at least one of the clinical symptoms thereof), to preventing or delaying the onset or development or progression of the disease or disorder.

The term "patient" or "subject" refers to a warm-blooded animal. In a most preferred embodiment, the subject or patient is human. It may be a human who has been diagnosed and is in the need of treatment for a disease or disorder, as disclosed herein.

The methods as defined herein may comprise administering an effective amount of a pharmaceutical combination to a subject in need. The term "effective amount" as defined herein is meant the administration of an amount of agent to an individual in need thereof, either in a single dose or as part of a series, that is effective for that elicitation, treatment or prevention. The effective amount will vary depending upon the health and physical condition of the individual to be treated, the taxonomic group of individual to be treated, the formulation of the combination, the assessment of the medical situation, and other relevant factors. It is expected that the amount will fall in a relatively broad range that can be determined through routine trials.

The terms "cancer" and "cancerous" refer to or describe the physiological condition in mammals that is typically characterized in part by unregulated cell growth. As used herein, the term "cancer" refers to non-metastatic and metastatic cancers, including early stage and late stage cancers. The term "precancerous" refers to a condition or a growth that typically precedes or develops into a cancer. By "non-metastatic" is meant a cancer that is benign or that remains at the primary site and has not penetrated into the lymphatic or blood vessel system or to tissues other than the primary site. Generally, a non-metastatic cancer is any cancer that is a Stage 0, I, or II cancer, and occasionally a Stage III cancer. By "early stage cancer" is meant a cancer that is not invasive or metastatic or is classified as a Stage 0, I, or II cancer. The term "late stage cancer" generally refers to a Stage III or Stage IV cancer, but can also refer to a Stage II cancer or a substage of a Stage II cancer. One skilled in the art will appreciate that the classification of a Stage II cancer as either an early stage cancer or a late stage cancer depends on the particular type of cancer. Illustrative examples of cancer include, but are not limited to, breast cancer, prostate cancer, ovarian cancer, cervical cancer, pancreatic cancer, colorectal cancer, lung cancer, hepatocellular cancer, gastric cancer, liver cancer, bladder cancer, cancer of the urinary tract, thyroid cancer, renal cancer, carcinoma, melanoma, brain cancer, non-small cell lung cancer, squamous cell cancer of the head and neck, endometrial cancer, multiple myeloma, rectal cancer, and esophageal cancer.

In an embodiment, the proliferative disease or disorder is associated with the up-regulation of the Wnt pathway.

In one embodiment, the proliferative disease or condition is selected from the group consisting of cancer, fibrosis, stem cell and diabetic retinopathy, rheumatoid arthritis, psoriasis and myocardial infarction.

In one embodiment, the proliferative disease or condition is selected from the group consisting of cervical cancer, colon cancer, colorectal cancer, breast cancer, bladder cancer, endometrial cancer, head and neck cancer, gastric cancer, lung cancer, ovarian cancer, prostate cancer, thyroid cancer, non-small-cell lung cancer, chronic lymphocytic leukemia, mesothelioma, melanoma, pancreatic adenocarcinoma, basal cell carcinoma, osteosarcoma, hepatocellular carcinoma, Wilm's tumour, medulloblastoma, pulmonary fibrosis, liver fibrosis, skin fibrosis, renal fibrosis, stem cell and diabetic retinopathy, rheumatoid arthritis, psoriasis and myocardial infarction.

In one embodiment, the proliferative disease or condition is lung cancer, prostate cancer, melanoma, osteosarcoma, colorectal cancer, or endometrial cancer.

In one embodiment, the proliferative disease or condition is colorectal cancer. In one embodiment, the proliferative disease or condition is a microsatellite stable colorectal cancer.

In one embodiment, the proliferative disease or condition is an endometrial cancer.

Dosing of the compound of Formula (I) can vary and may occur at intervals of minutes, hours, days, weeks, months or years or continuously over any one of these periods. In one embodiment, the method comprises administering the compound of Formula (I) once every day to the subject. **In** one embodiment, the method comprises administering the compound of Formula (I) once every two days (i.e. once every other day). In one embodiment, the method comprises administering the compound of Formula (I) once every three, four, five, six or seven days. In one embodiment, the method comprises administering the compound of Formula (I) twice or three times a day. The method may comprises administering the compound of Formula (I) orally to the subject. The compound of Formula (I) may be administered in a single dose or a series of doses. The compound of Formula (I) may also be administered as a discrete unit or a combination of discrete units.

The dosing schedule of the anti-PD-1 antibody molecule can vary from e.g., once a week to once every 2, 3, or 4 weeks. In one embodiment, the method comprises administering the anti-PD-1 antibody molecule once every 21 days to the subject. The method may comprise administering the anti-PD-1 antibody molecule via intravenous infusion to the subject. The method may comprise administering the anti-PD-1 antibody molecule via intravenous injection to the subject.

The compound of Formula (I) may be administered at a dose of about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 38 mg, about 39 mg, about 40 mg, about 41 mg, about 42 mg, about 43 mg, about 44 mg, about 45 mg, about 46 mg, about 47 mg, about 48 mg, about 49 mg or about 50 mg. In one embodiment, the compound of Formula (I) is administered at a dose of about 24 mg to the subject. In one embodiment, the compound of Formula (I) is administered at a dose of about 16 mg to the subject. In one embodiment. the compound of Formula (I) is administered at a dose of about 8 mg to the subject. In one embodiment, the compound of Formula (I) is administered at a dose of about 4 mg to the subject.

The anti-PD-1 antibody molecule may be administered at a dose of about 10 mg, about 20mg, about 30 mg, about 40 mg, about 50mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg or about 400 mg. In one embodiment, the anti-PD-1 antibody molecule is administered at a dose of about 200 mg to the subject.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1 %, 0.05%, or 0.01% of the stated value. Unless otherwise clear from context, all numerical values provided herein can be modified by the term about.

Suitable dosage amounts and dosing regimens can be determined by the attending physician and may depend on the severity of the condition as well as the general age, health and weight of the patient to be treated. For example, the dosing schedule (such as the dosing schedule of the compound of Formula (I)) can be altered during the course of treatment such that the dosage or frequency is increased or reduced during the course of treatment. The dosing schedule may also include breaks in administration.

In one embodiment, the method further comprises administering a bone protective agent. The bone protective agent may be denosumab or zoledronic acid or both.

The antibody molecules can be administered by a variety of methods known in the art, although for many therapeutic applications, the preferred route/mode of administration is intravenous injection or infusion. For intravenous injection or infusion, therapeutic compositions typically should be sterile and stable under the conditions of manufacture and storage. The composition can be formulated as a solution, microemulsion, dispersion, liposome, or other ordered structure suitable to high antibody concentration. Sterile injectable solutions can be prepared by incorporating the active compound (i.e., antibody or antibody portion) in the required amount in an appropriate solvent with one or a combination of ingredients as required, followed by filtered sterilization. Generally, dispersions are prepared by incorporating the active compound into a sterile vehicle that contains a basic dispersion medium and the required other ingredients. In the case of sterile powders for the preparation of sterile injectable solutions. the preferred methods of preparation are vacuum drying and freeze-drying that yields a powder of the active ingredient plus any additional desired ingredient from a previously sterile- filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

It would be understood that the route and/or mode of administration will vary depending upon the desired results. For example, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art (e.g. Sustained and Controlled Release Drug Delivery Systems, J. R. Robinson, ed., Marcel Dekker, Inc., New York, 1978).

Disclosed herein is a pharmaceutical combination as defined herein for use as a medicament.

Disclosed herein is a pharmaceutical combination as defined herein for use in the treatment of a proliferative disease or disorder associated with Wnt pathway in a subject.

Disclosed herein is the use of a pharmaceutical combination as defined herein in the manufacture of a medicament for the treatment of a proliferative disease or disorder associated with Wnt pathway in a subject.

Disclosed herein is the use of a compound of Formula (I) or a pharmaceutically acceptable salt or solvate thereof in the manufacture of a medicament for the treatment of a proliferative disease or disorder associated with Wnt pathway in a subject, wherein the medicament is to be administered with an anti-PD-1 antibody molecule or a pharmaceutically acceptable salt or solvate thereof to the subject in need.

Disclosed herein is the use of an anti-PD-1 antibody molecule or a pharmaceutically acceptable salt or solvate thereof in the manufacture of a medicament for the treatment of a proliferative disease or disorder associated with Wnt pathway in a subject, wherein the medicament is to be administered with a compound of Formula (I) or a pharmaceutically acceptable salt or solvate thereof to the subject in need.

Provided herein are pharmaceutical compositions comprising the pharmaceutical combination as disclosed herein. In one embodiment, there is provided a pharmaceutical composition comprising a compound of Formula (I) and an anti-PD-1 antibody. In one embodiment, there is provided a pharmaceutical composition comprising a compound of Formula (I) and a pharmaceutical composition comprising an anti-PD-1 antibody.

The compositions may contain any suitable carriers, diluents or excipients. These include all conventional solvents, dispersion media, fillers, solid carriers, coatings, antifungal and antibacterial agents, dermal penetration agents, surfactants, isotonic and absorption agents and the like. It will be understood that the compositions of the invention may also include other supplementary physiologically active agents.

The carrier must be pharmaceutically "acceptable" in the sense of being compatible with the other ingredients of the composition and not injurious to the subject. Compositions include those suitable for oral, rectal, nasal, topical (including buccal and sublingual), vaginal or parental (including subcutaneous, intramuscular, intravenous and intradermal) administration. The compositions may conveniently be presented in unit dosage form and may be prepared by any methods well known in the art of pharmacy. Such methods include the step of bringing into association the active ingredient with the carrier which constitutes one or more accessory ingredients. In general, the compositions are prepared by uniformly and intimately bringing into association the active ingredient with liquid carriers or finely divided solid carriers or both, and then if necessary shaping the product.

Compositions of the present invention suitable for oral administration may be presented as discrete units such as capsules, sachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. inert diluent, preservative disintegrant (e.g. sodium starch glycolate, cross-linked polyvinyl pyrrolidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Compositions suitable for topical administration in the mouth include lozenges comprising the active ingredient in a flavoured base, usually sucrose and acacia or tragacanth gum; pastilles comprising the active ingredient in an inert basis such as gelatine and glycerin, or sucrose and acacia gum; and mouthwashes comprising the active ingredient in a suitable liquid carrier.

Compositions suitable for topical administration to the skin may comprise the compounds dissolved or suspended in any suitable carrier or base and may be in the form of lotions, gel, creams, pastes, ointments and the like. Suitable carriers include mineral oil, propylene glycol, polyoxyethylene, polyoxypropylene, emulsifying wax, sorbitan monostearate, polysorbate 60, cetyl esters wax, cetearyl alcohol, 2-octyldodecanol, benzyl alcohol and water. Transdermal patches may also be used to administer the compounds of the invention.

Compositions for rectal administration may be presented as a suppository with a suitable base comprising, for example, cocoa butter, glycerin, gelatine or polyethylene glycol.

Compositions suitable for vaginal administration may be presented as pessaries, tampons, creams, gels, pastes, foams or spray formulations containing in addition to the active ingredient such carriers as are known in the art to be appropriate.

Compositions suitable for parenteral administration include aqueous and non-aqueous isotonic sterile injection solutions which may contain anti-oxidants, buffers, bactericides and solutes which render the composition isotonic with the blood of the intended recipient; and aqueous and non-aqueous sterile suspensions which may include suspending agents and thickening agents. The compositions may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage compositions are those containing a daily dose or unit, daily sub-dose, as herein above described, or an appropriate fraction thereof, of the active ingredient.

It should be understood that in addition to the active ingredients particularly mentioned above, the compositions of this invention may include other agents conventional in the art having regard to the type of composition in question, for example, those suitable for oral administration may include such further agents as binders, sweeteners, thickeners, flavouring agents disintegrating agents, coating agents, preservatives, lubricants and/or time delay agents. Suitable sweeteners include sucrose, lactose, glucose, aspartame or saccharine. Suitable disintegrating agents include cornstarch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Throughout this specification and the statements which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in the subject specification, the singular forms "a", "an" and "the" include plural aspects unless the context clearly dictates otherwise. Thus, for example, reference to a "formulation" includes a single formulation, as well as two or more formulations; reference to "an agent" includes a single agent, as two or more agents; reference to "the disclosure" includes a single and multiple aspects taught by the disclosure; and so forth.

Certain embodiments of the invention will now be described with reference to the following examples which are intended for the purpose of illustration only.

### EXAMPLES

### Example 1

### Preparation of compound of Formula (I)

### Synthesis of amines

Suzuki Method A: A stirred solution of the arylhalide (1 equiv.), boronic acid (1.5 equiv.) and sodium carbonate (2 equiv.) in toluene (0.08 M) and water (0.32 M) was degassed for 15 min with argon. Tetrakis(triphenylphosphine)palladium(0) (0.05 equiv.) was added to reaction mixture and the reaction mixture was heated to reflux for 16 h. After completion of starting material, the reaction mixture was concentrated and water was added to reaction mixture and extracted with ethyl acetate. The combined organic layers were washed with brine, dried with Na₂SO₄ and concentrated under vacuum. The crude compound was purified by column chromatography to afford the purified product.

### Synthesis of 6-phenylpyridazin-3-amine.

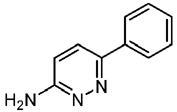

¹H NMR (400 MHz, DMSO-d₆) δ (ppm): 7.96-7.94 (d, J=8 Hz, 2H), 7.82-7.80 (d, J=9.2 Hz, 1H), 7.48-7.35 (m, 3H), 6.86-6.84 (m, 1H), 6.64(br s, 2H). LC-MS: m/z 172.0 (M+H) with a purity of 82%.

Amide coupling Method A: To a stirred solution of commercially available 2-(1,3-dimethyl-2,6-dioxo-2,3-dihydro-1H-purin-7(6H)-yl)acetic acid, 1 (1 equiv.) in dichloromethane (0.01 M) was added HATU (1.3 equiv.), triethylamine (1.5 equiv.) and the respective amine (1 equiv.). The reaction mixture was allowed to stir at room temperature. Upon completion of the reaction, water was added to the reaction mixture and the mixture was extracted with dichloromethane. The organic layer was washed with brine solution, dried over anhydrous Na₂SO₄, and concentrated under vacuum to afford the crude product. The crude product is further purified by column chromatography.

### 2-(1,3-dimethyl-2,6-dioxo-1,2,3,6-tetrahydro-7H-purin-7-yl)-N-(6-phenylpyridazin-3-yl)acetamide (alternatively named 2-(1,3-dimethyl-2,6-dioxo-2,3-dihydro-lH-purin-7(6H)-yl)-N-(6-phenyl pyridazin-3-yl)acetamide).

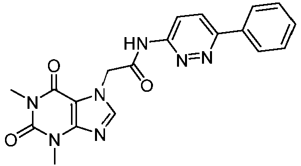

¹H NMR (400 MHz, DMSO-d₆) δ (ppm): 11.77 (s, 1H), 8.32-8.25 (m, 2H), 8.12-8.10 (m, 3H), 7.57-7.49 (m, 3H), 5.37 (s, 2H), 3.46 (s, 3H), 3.19 (s, 3H). LC-MS: m/z 390.1 (M+H) with a purity of 99%.

### Example 2

### Single agent activity of compound A increased cell surface PD-1 expression and upregulation of genes making the tumor more "hot"

FFPE tumor tissue of patient 202-101 with unknown primary tumour (presumably cholangiocarcinoma) was prepared from paired biopsies, pre-dose and on C1D17 post-dose. Sections were assayed via IHC/OPAL^{™} and analysed on the Vectra3 (Perkin Elmer). Cells were counted on the Vectra3 in each high-power field at a 400X magnification and means ±SD were graphed from a minimum of n=3 different fields. The ratio of CD8⁺ to FOXP3⁺ and CD4⁺ to FOXP3⁺ cells was plotted with numbers determined from the single stainings (Figure 1).

### Compound A as a single agent changed immune environment gene expression levels to make tumor more hot

Nanostring analysis of FFPE and frozen tumor tissues from the patient with unknown primary (presumably cholangiocarcinoma) 202-101 (T4-T3) and the MSS CRC 102-102 (T2-T1) patient (bearing an RSPO gene fusion) using the Pan-Cancer immune panel (Figure 2A). Grouped analysis showed similar pre- and post-dose changes in both samples. (Figure 2B) Most up- and downregulated genes common for both cancer types are shown (Figure 2C and 2D). Shows the most up and down-regulated genes in the CRC patient with RSPO fusion and the patient with unknown primary tumour (presumed to be cholangiocarcinoma), respectively. Red gene symbols indicate genes that are up- or down-regulated in both of the patients.

### Example 3

### Dose Escalation Study

A 3+3 dose escalation study of compound A with the standard dose of pembrolizumab will first be tested. In this study, compound A at different doses (e.g. 8 mg, 16 mg, or 24 mg) will be administrated simultaneously with pembrolizumab (e.g. 200 mg) to determine the recommended dose (RD) of compound A for dose expansion.

### Example 4

### Dose Expansion Study

### Primary Objectives

To assess the safety of compound A administered orally every other day at the recommended does (RD) of 16 mg (which may be adjusted to 8 mg or 24 mg depending the outcome of the dose escalation study) as a single agent until disease progression and then in combination with pembrolizumab (PD-1 inhibitor), as measured by monitoring of adverse events (AEs), imaging assessments, 12-lead electrocardiogram readings, Eastern Cooperative Oncology Group performance status, clinical laboratory test results, and vital sign measurements in two groups of patients with advanced or metastatic, or unresectable solid malignancies that are refractory, intolerant or not suitable for available treatments according to the treating physician.

To assess the tolerability of compound A administered orally every other day at the RD of 16 mg as a single agent and in combination with pembrolizumab (PD-1 inhibitor). The tolerability assessment will be made by monitoring of fatigue and gastrointestinal symptoms, and when applicable, immuno-related side effects in two groups of patients (Group 1 and Group 2 [subgroups 2a, 2b, and 2c]) with advanced or metastatic, or unresectable solid malignancies that are refractory, intolerant or not suitable for available treatment according to the treating physician.

### Patient Population

Recommended dose of compound A (compound of Formula (I)) as a single agent until progression and then in combination with pembrolizumab (PD-1 inhibitor), with or without bone protective treatment (denosumab and subsequently zoledronic acid if no response is observed with denosumab). Patients with advanced or metastatic, or unresectable solid malignancies that are refractory, intolerant or not suitable for available treatment according to the treating physician, will be divided into two groups.

**Group 1** will consist of up to 20 patients with MSS colorectal cancer (CRC) who will be prospectively selected for the presence of one of the four different recurrent gene fusions involving R-spondin (RSPO)2 or RSPO3 (Fusions A-D), using the D3 RSPO Gene Fusion Test.

**Group 2** will consist of up to 45 patients divided in three subgroups;
- subgroup 2a (N=15): patients with MSS-CRC who will be prospectively selected for the absence of all four different recurrent gene fusions involving RSPO2 or RSPO3 (Fusions A-D), using the D3 RSPO Gene Fusion Test;
- subgroup 2b (N=15): patients with MSS ovarian cancer who will be recruited without prospective selection (for additional mutations); and
- subgroup 2c (N=15): patients with MSS endometrial cancer who will be recruited without prospective selection (for additional mutations); .

### Study Design

Preliminary efficacy and safety data will be collected during treatment with the recommended dose of 16 mg compound A administered orally once every other day as a single agent, and following progression, in combination with pembrolizumab (PD-1 inhibitor) with or without bone protective treatment (denosumab and subsequently zoledronic acid if no response is observed with denosumab) in an open-label, non-randomised study. The completion of two cycles (6 weeks) of compound A monotherapy before adding pembrolizumab treatment is recommended. However, the completion of a minimum of 1 cycle (3 weeks) of compound A monotherapy before adding pembrolizumab is acceptable. Patients that do not complete the minimum duration of 1 cycle (21 days) of compound A monotherapy for reasons other than safety, may be replaced. Actual dosing schedule and frequency of bone protective treatment will be based on observed serum levels of the bone resorption biomarker (β-CTX).

### Duration

Single agent treatment period (Dose expansion) - administration of compound A until disease progression as per RECIST Version 1.1 (if the patient does not enter the pembrolizumab combination treatment phase), start of a new cancer therapy, or completion of 12 cycles of treatment (1 cycle: 21 days).

Combination treatment period - administration of compound A and pembrolizumab until disease progression as per iRECIST, start of a new cancer therapy or completion of 12 cycles of treatment (1 cycle: 21 days). In both, single agent or combination treatment periods, so long as the sponsor agrees, patients who complete 12 cycles of treatment (1 cycle: 21 days) may continue until disease progression, unacceptable toxicity, patient withdrawal, or loss of clinical benefit.

### Study Drug, Dosage, and Route of Administration (Dose expansion)

**All** patients will receive the recommended dose of 16 mg of the compound A as a single agent until disease progression, and then in combination with pembrolizumab until confirmed disease progression or loss of clinical benefit. Pembrolizumab will be administered at the dose of 200 mg (30 minutes intravenous infusion) every 21 days. Any time during the study, compound A may be reduced to 8 mg every other day if not tolerated.

### Bone Protection Treatment, Dosage, and Route of Administration:

Denosumab will be administered at a starting dose of 120 mg SC every 28 days when β-CTX serum levels are >1000 pg/mL. If after 1 week of denosumab (SC) treatment β-CTX levels do not decrease to < 1000 pg/mL, denosumab will be discontinued and zoledronic acid will be started at the dose of 4 mg intravenously every 3-4 weeks. If after denosumab and zoledronic acid administration (in sequence) β-CTX levels do not decrease <1000 pg/mL within 3 weeks (3 consecutive assessments), the patient will be discontinued from the study. During denosumab or zoledronic acid treatment, patients will also receive calcium 1000 mg or higher orally daily, based on the investigator's clinical judgement, and vitamin D (at least 400 IU) orally daily.

## Claims

1. A pharmaceutical combination, comprising:
a) a compound of Formula (I) having the following structure: 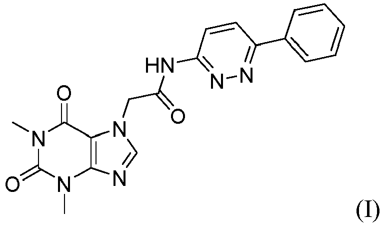 or a pharmaceutically acceptable salt or solvate thereof; and
b) an anti-PD-1 antibody molecule or a pharmaceutically acceptable salt or solvate thereof.

2. The pharmaceutical combination according to claim 1, wherein the anti-PD-1 antibody molecule is Pembrolizumab or a fragment thereof.

3. A pharmaceutical combination according to claim 1 for use in the treatment of a proliferative disease or disorder associated with Wnt pathway in a subject.

4. The pharmaceutical combination for use according to claim 3, wherein the proliferative disease or condition is cancer, fibrosis, stem cell and diabetic retinopathy, rheumatoid arthritis, psoriasis or myocardial infarction.

5. The pharmaceutical combination for use according to claim 3 or claim 4, wherein the proliferative disease or condition is lung cancer, prostate cancer, melanoma, osteosarcoma, colorectal cancer or endometrial cancer.

6. The pharmaceutical combination for use according to claim 3 or claim 4, wherein the proliferative disease or condition is selected from the group consisting of cervical cancer, colon cancer, breast cancer, bladder cancer, head and neck cancer, gastric cancer, lung cancer, ovarian cancer, prostate cancer, thyroid cancer, non-small-cell lung cancer, chronic lymphocytic leukemia, mesothelioma, melanoma, pancreatic adenocarcinoma, basal cell carcinoma, osteosarcoma, hepatocellular carcinoma, Wilm's tumour, medulloblastoma, pulmonary fibrosis, liver fibrosis, skin fibrosis, renal fibrosis, stem cell and diabetic retinopathy, rheumatoid arthritis, psoriasis and myocardial infarction.

7. The pharmaceutical combination for use according to any one of claims 3-6, wherein the compound of Formula (I) is to be administered once every day to the subject.

8. The pharmaceutical combination for use according to any one of claims 3-7, wherein the anti-PD-1 antibody molecule is to be administered once every 21 days to the subject.

9. The pharmaceutical combination for use according to any one of claims 3-8, wherein the compound of Formula (I) is to be administered orally to the subject.

10. The pharmaceutical combination for use of any one of claims 3-9, wherein the anti-PD-1 antibody molecule is to be administered via intravenous infusion to the subject.

11. The pharmaceutical combination for use according to any one of claims 3-10, wherein the compound of Formula (I) is to be administered at a dose of about 8 mg, about 16 mg or about 24 mg to the subject.

12. The pharmaceutical combination for use according to any one of claims 3-11, wherein the anti-PD-1 antibody molecule is to be administered at a dose of about 200 mg to the subject.

## Patentansprüche

1. Pharmazeutische Kombination, umfassend:
a) eine Verbindung der Formel (I) mit der folgenden Struktur: 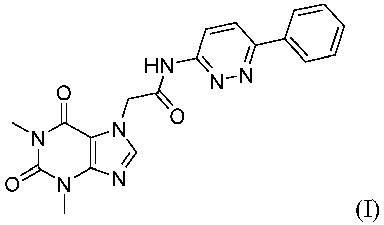 oder ein pharmazeutisch unbedenkliches Salz oder Solvat davon; und
b) ein Anti-PD-1-Antikörpermolekül oder ein pharmazeutisch unbedenkliches Salz oder Solvat davon.

2. Pharmazeutische Kombination nach Anspruch 1, wobei es sich bei dem Anti-PD-1-Antikörpermolekül um Pembrolizumab oder ein Fragment davon handelt.

3. Pharmazeutische Kombination nach Anspruch 1 zur Verwendung bei der Behandlung einer proliferativen Krankheit oder Störung, die mit dem Wnt-Signalweg in einem Individuum assoziiert ist.

4. Pharmazeutische Kombination zur Verwendung nach Anspruch 3, wobei es sich bei der proliferativen Krankheit oder Störung um Krebs, Fibrose, Stamzellen- und diabetische Retinopathie, rheumatoide Arthritis, Psoriasis oder Myokardinfarkt handelt.

5. Pharmazeutische Kombination zur Verwendung nach Anspruch 3 oder Anspruch 4, wobei es sich bei der proliferativen Krankheit oder Störung um Lungenkrebs, Prostatakrebs, Melanom, Osteosarkom, Kolorektalkrebs oder Endometriumkrebs handelt.

6. Pharmazeutische Kombination zur Verwendung nach Anspruch 3 oder Anspruch 4, wobei die proliferative Krankheit oder Störung aus der Gruppe bestehend aus Gebärmutterhalskrebs, Darmkrebs, Brustkrebs, Blasenkrebs, Kopf-Hals-Krebs, Magenkrebs, Lungenkrebs, Eierstockkrebs, Prostatakrebs, Schilddrüsenkrebs, nicht kleinzelligem Lungenkrebs, chronischer lymphozytischer Leukämie, Mesotheliom, Melanom, Bauchspeicheldrüsen-Adenokarzinom, Basalzellkarzinom, Osteosarkom, hepatozellulärem Karzinom, Wilms-Tumor, Medulloblastom, Lungenfibrose, Leberfibrose, Hautfibrose, Nierenfibrose, Stamzellen- und diabetischer Retinopathie, rheumatoider Arthritis, Psoriasis und Myokardinfarkt ausgewählt ist.

7. Pharmazeutische Kombination zur Verwendung nach einem der Ansprüche 3-6, wobei die Verbindung der Formel (I) dem Individuum einmal am Tag verabreicht wird.

8. Pharmazeutische Kombination zur Verwendung nach einem der Ansprüche 3-7, wobei das Anti-PD-1-Antikörpermolekül dem Individuum einmal alle 21 Tage verabreicht wird.

9. Pharmazeutische Kombination zur Verwendung nach einem der Ansprüche 3-8, wobei die Verbindung der Formel (I) dem Individuum oral verabreicht wird.

10. Pharmazeutische Kombination zur Verwendung nach einem der Ansprüche 3-9, wobei das Anti-PD-1-Antikörpermolekül dem Individuum mittels intravenöser Infusion verabreicht wird.

11. Pharmazeutische Kombination zur Verwendung nach einem der Ansprüche 3-10, wobei die Verbindung der Formel (I) dem Individuum in einer Dosis von etwa 8 mg, etwa 16 mg oder etwa 24 mg verabreicht wird.

12. Pharmazeutische Kombination zur Verwendung nach einem der Ansprüche 3-11, wobei das Anti-PD-1-Antikörpermolekül dem Individuum in einer Dosis von etwa 200 mg verabreicht wird.

## Revendications

1. Combinaison pharmaceutique, comprenant :
a) un composé de formule (I) ayant la structure suivante : 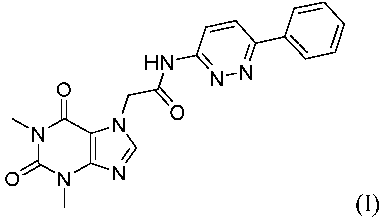 ou sel ou solvate pharmaceutiquement acceptable de ce composé ; et
b) une molécule d'anticorps anti-PD-1 ou un sel ou solvate pharmaceutiquement acceptable de celle-ci.

2. Combinaison pharmaceutique selon la revendication 1, dans laquelle la molécule d'anticorps anti-PD-1 est le pembrolizumab ou un fragment de celui-ci.

3. Combinaison pharmaceutique selon la revendication 1 pour une utilisation dans le traitement d'une maladie proliférative ou d'un trouble associé à la voie Wnt chez un sujet.

4. Combinaison pharmaceutique pour utilisation selon la revendication 3, dans laquelle la maladie ou l'affection proliférative est le cancer, la fibrose, les cellules souches et la rétinopathie diabétique, la polyarthrite rhumatoïde, le psoriasis ou l'infarctus du myocarde.

5. Combinaison pharmaceutique pour utilisation selon la revendication 3 ou la revendication 4, dans laquelle la maladie ou l'affection proliférative est le cancer du poumon, le cancer de la prostate, le mélanome, l'ostéosarcome, le cancer colorectal ou le cancer de l'endomètre.

6. Combinaison pharmaceutique pour une utilisation selon la revendication 3 ou la revendication 4, dans laquelle la maladie ou l'affection proliférative est choisie dans le groupe constitué par un cancer du col de l'utérus, un cancer du côlon, un cancer du sein, un cancer de la vessie, un cancer de la tête et du cou, un cancer de l'estomac, un cancer du poumon, un cancer de l'ovaire, un cancer de la prostate, un cancer de la thyroïde, un cancer du poumon non à petites cellules, une leucémie lymphoïde chronique, un mésothéliome, un mélanome, un adénocarcinome pancréatique, un carcinome basocellulaire, un ostéosarcome, un carcinome hépatocellulaire, une tumeur de Wilms, un médulloblastome, une fibrose pulmonaire, une fibrose hépatique, une fibrose cutanée, une fibrose rénale, une rétinopathie diabétique et à cellules souches, une polyarthrite rhumatoïde, un psoriasis et un infarctus du myocarde.

7. Combinaison pharmaceutique pour utilisation selon l'une quelconque des revendications 3 à 6, dans laquelle le composé de formule (I) doit être administré une fois par jour au sujet.

8. Combinaison pharmaceutique pour utilisation selon l'une quelconque des revendications 3 à 7, dans laquelle la molécule d'anticorps anti-PD-1 doit être administrée une fois tous les 21 jours au sujet.

9. Combinaison pharmaceutique pour utilisation selon l'une quelconque des revendications 3 à 8, dans laquelle le composé de formule (I) doit être administré par voie orale au sujet.

10. Combinaison pharmaceutique pour utilisation de l'une quelconque des revendications 3 à 9, la molécule d'anticorps anti-PD-1 devant être administrée par perfusion intraveineuse au sujet.

11. Combinaison pharmaceutique pour utilisation selon l'une quelconque des revendications 3 à 10, dans laquelle le composé de formule (I) doit être administré à une dose d'environ 8 mg, environ 16 mg ou environ 24 mg au sujet.

12. Combinaison pharmaceutique pour utilisation selon l'une quelconque des revendications 3 à 11, la molécule d'anticorps anti-PD-1 devant être administrée à une dose d'environ 200 mg au sujet.
